# EUROPEAN PATENT APPLICATION

(11) **EP 4 268 807 A1**
(43) Date of publication of application: **01.11.2023**
(21) Application number: 21911485.7
(22) Date of filing: 21.12.2021
(51) Int. Cl.: A61K 9/51, A61K 47/12, A61K 9/00, A61K 33/10, A61P 3/04, A61K 8/02, A61K 8/36, A61K 8/19

(54) **FATTY ACID-MODIFIED POLYMER NANOPARTICLES, AND USE THEREOF**

(30) Priority: 24.12.2020 KR 20200183085
(71) Applicant: Supernova Bio Co., Ltd., Seoul 04385 (KR)
(72) Inventor: LEE, Kuen Yong, Seoul 06084 (KR); LEE, Hye Won, Seoul 03782 (KR)
(74) Representative: Holt, Lucy Rose
(86) International application number: PCT/KR2021/019527
(87) International publication number: WO 2022/139418

(57) **Abstract**

The present invention relates to fatty acid-modified polymer nanoparticles, and a use thereof.

In the present invention, nanoparticles having embedded calcium carbonate crystals can be formed using a biocompatible polymer and an adipocyte-targeting ligand (a fatty acid) to minimize delivery to surrounding cells and tissues other than adipocytes and maximize the embedding of the nanoparticles into adipocytes.

The nanoparticles according to the present invention can be produced as an injectable preparation, and can be applied to local lipolysis supplements or diet and beauty products that break down localized fat.

## Description

### [Technical Field]

The present invention relates to fatty acid-introduced polymer nanoparticles, and a use thereof.

### [Background Art]

The reduction of subcutaneous fat existing under the epidermis and dermis of the skin is one of the most important fields of cosmetic treatment, and various treatment methods are used for the purpose of such cosmetic treatment.

The treatment for reducing subcutaneous fat includes: liposuction inhaling fat by inserting a cannula into subcutaneous fat, refrigeration procedures for cooling and necrosis of subcutaneous fat by attaching a cooling pad to a skin surface, thermal heating procedures for heating and removing subcutaneous fat by irradiating high frequency or ultrasonic waves to subcutaneous fat tissue, carboxytherapy procedures for removing fat by slowly injecting carbon dioxide (CO₂) into subcutaneous fat through an injection needle to promote blood flow circulation and lymph circulation of adipose tissue, mesotherapy procedures that inject a drugs for obesity treatment into subcutaneous fat, and the like.

The liposuction, which is known to be the most effective among the procedures, has disadvantages in pain accompanying the procedure and future management. Typically, there is bleeding during liposuction and pain during the procedure. Even after the procedure, pain may be caused, and there may be cases in which painkillers have to be taken depending on the individual. In addition, a compression garment must be worn for at least a week after inhalation surgery, and care is required for about a month after the procedure.

The refrigeration procedure is simple to perform, but has a disadvantage in that the treatment effect is low. Korean Patent Laid-Open Publication No. 10-2011-0119640 uses an invasive procedure in which a probe cooled by circulating a refrigerant inside is inserted into subcutaneous fat. However, in the case of the invasive cooling procedure, although the procedure time is shorter than that of the non-invasive cooling procedure, there is the disadvantage of requiring a considerably long procedure time to prevent necrosis of subcutaneous fat by cooling.

Meanwhile, the carboxytherapy is a procedure that intensively treats areas where fat is excessively accumulated, and Korean Registered Patent No. 10-0772961 increased an efficiency of fat removal by performing both mesotherapy and carboxytherapy procedures. However, in the above patent, since a separate syringe needle is used for each procedure, there is a disadvantage in that the internal structure is complicated and a separate incision is made by each needle.

Currently, local lipolysis supplements that have been approved by the Ministry of Food and Drug Safety have very limited uses, and off-label procedures are frequently performed in the market. The off-label procedures lack the basis for safety and effectiveness, are in a blind spot of safe use management because they are non-benefit areas, and lack institutional management.

As a drug that has been approved as a supplement for lipolysis, there is Belkyra, which kills adipocytes by destroying the cell membrane of local adipocytes. However, the Belkyra has the disadvantage that it is limitedly available only for double chin surgery. In addition, it is currently reported that this drug non-specifically destroys cell membranes and has a great effect on surrounding cells as well as fat cells, and thus, may have side effects on surrounding tissues, thereby increasing a risk of breast cancer or colon cancer.

### [Disclosure]

### [Technical Problem]

In order to solve the above-mentioned problems, the present invention is to provide polymer nanoparticles that decompose local fat and uses thereof.

### [Technical Solution]

The present invention provides a nanoparticle comprising: a calcium carbonate crystal, a biocompatible polymer and a fatty acid, wherein the calcium carbonate is embedded in the nanoparticle, and the fatty acid is exposed on the surface of the nanoparticle.

In the case of existing local lipolysis procedures, treatment is performed by injecting a drug directly into the area where the fat has been accumulated with an injection needle, whereby there is a possibility of destroying other cells around the injection site as well because non-specific drugs are used, and there was a risk of numbness or nerve damage if a nearby nerve was touched. In order to solve these problems, the present inventors have found that by using nanoparticles including a calcium carbonate crystal, a biocompatible polymer and a fatty acid, the nanoparticles can be specifically introduced into adipocytes by the fatty acid exposed on the surface of the nanoparticles, and completed the present invention.

As used herein, the term "calcium carbonate crystal" is distinguished from precipitated calcium carbonate produced by chemical processing, and generally refers to a product prepared by mechanically grinding and classifying high-purity crystalline limestone.

The nanoparticles of the present invention refer to a solid colloidal structure which have a form in which the calcium carbonate crystals are embedded in a biocompatible polymer, this is, a form in which the calcium carbonate crystals are embedded non-fluidly in a hard-shell spherical structure formed by the biocompatible polymers, wherein the calcium carbonate crystals are evenly embedded without significant difference in distribution probability from the center to the outer surface of the spherical structure.

The nanoparticles of the present invention cause a reaction in which the calcium carbonate crystals embedded in the particles generate carbon dioxide gas in an acidic environment. The nanoparticles of the present invention do not have a core-shell form containing the calcium carbonate inside a layered membrane structure, but a form in which the calcium carbonate crystals are embedded in an inside-filled solid crystal. That is, unlike a structure in which gas generated inside leaks out at the same time as gas is generated, when the calcium carbonate crystals embedded in the nanoparticles generate carbon dioxide gas, the internal pressure of the nanoparticles gradually increases, finally leading to explosion of the nanoparticles. Due to this characteristic, the effects of cell strike by the release of carbon dioxide gas droplets and the explosion of nanoparticles are simultaneously achieved.

In one embodiment of the present invention, the biocompatible polymer of the present invention is surface-modified with a fatty acid. Specifically, the fatty acid is mixed with the biocompatible polymer to form the spherical structure, wherein the fatty acid is in a form exposed on the surface of the structure. The fatty acid of the present invention may include saturated fatty acids and unsaturated fatty acids, and preferably may be C₁₂ to C₂₀ saturated or unsaturated fatty acids. The fatty acids exposed on the surface of the nanoparticles of the present invention have adipocyte-targeting directivity. Specifically, the fatty acid on the surface of the nanoparticles reacts with fatty acid transporter proteins present in the cell membrane of adipocytes so that the nanoparticles are introduced into the adipocytes by means of intracellular absorption. Therefore, the nanoparticles surface-modified with fatty acids can be selectively absorbed/accumulated into adipose cells or adipose tissues, and generate carbon dioxide gas within the adipose cells to transform or destroy the structure of nanoparticles so that the gas is rapidly ejected, which can physically strike the adipose cells to decompose them or to cause their necrotic necrosis, thereby reducing the adipose tissues.

The biocompatible polymer of the present invention refers to a polymer having tissue compatibility and blood compatibility that does not destroy tissue or coagulate blood by contact with living tissue or blood, and can be appropriately selected without any particular limitation as long as it can form a solid structure in which the calcium carbonate crystals are embedded by the emulsion method of the present invention.

In one embodiment of the present invention, the biocompatible polymer of the present invention is a polymer having a polylactide (PLA), polyglycolide (PGA), polylactide-polyglycolide copolymer (PLGA), starch, glycogen, chitin, peptidoglycan, lignosulfonate, tannic acid, lignin, pectin, polyethylene glycol, polyethylene oxide, polyvinyl alcohol, polyethylene oxide-polypropylene oxide block copolymer, cellulose, hemicellulose, heparin, hyaluronic acid, dextran or alginate structure. Preferably, polylactide-polyglycolide copolymer (PLGA) may be used.

In one embodiment of the present invention, the diameter of the nanoparticles of the present invention may be 100 to 300 nm, preferably 120 to 290 nm, more preferably 150 to 280 nm, even more preferably 200 to 260 nm. Within the above diameter range, the cell striking effect capable of inducing the necrotic death of adipocytes can be maximized.

In one embodiment of the present invention, the weight ratio of the fatty acid to the biocompatible polymer may be 0.01: 1 to 0.5:1 (fatty acid: polymer), preferably 0.05:1 to 0.15:1. Within this weight ratio, targeting to adipocytes and cell viability can be increased. When the content of the fatty acid is greater than the above range, the diameter of the nanoparticles may be increased, but a lot of loss may occur due to a low temperature in the manufacturing process, and the stability of the manufactured nanoparticles may be degraded.

In one embodiment of the present invention, the weight ratio of the calcium carbonate crystals to the biocompatible polymer may be 0.001:1 to 1:1 (calcium carbonate: polymer). Preferably, the weight ratio of the calcium carbonate crystals to the biocompatible polymer is 0.01:1 to 0.8:1, more preferably 0.1:1 to 0.6:1. The calcium carbonate crystals and the biocompatible polymer having the above weight ratio can maximize the effect of reducing the adipocytes. The ratio of the calcium carbonate crystals to the biocompatible polymer does not significantly affect the diameter of the gas-generating nanoparticles produced. As shown in Table 2, as the content of the calcium carbonate crystals increases, the content efficiency decreases significantly, whereby it is difficult to expect a content higher than the above range, and in the case of a higher content, the hard-shell structure by the biocompatible polymer may also be relatively weak. On the other hand, when the calcium carbonate is included less than the above range, the amount of gas generated may be insufficient to obtain the effect of striking the adipocytes.

In one embodiment of the present invention, when using the nanoparticles prepared within the above weight ratio of the calcium carbonate, the biocompatible polymer and the fatty acid, a significant fat reduction effect was induced *in vitro.*

According to one aspect of the present invention, the present invention provides a composition for lipolysis, comprising the above-described nanoparticles. The composition for lipolysis according to the present invention has an effect of specifically releasing carbon dioxide in the environment inside the adipocytes, and unlike the conventional local lipolysis treatment, adipocyte tissue-targeting is possible.

According to one aspect of the present invention, the present invention provides a pharmaceutical composition for preventing or treating obesity, comprising the above-described nanoparticles and/or a composition comprising the same.

According to one aspect of the present invention, the present invention provides a method for producing nanoparticles, comprising the steps of:
(a) mixing a first water phase containing calcium carbonate crystals with an oil phase containing fatty acids and biocompatible polymers to form a water-in-oil type single emulsion (w/o);
(b) mixing the emulsion of step (a) with a second water phase to form a water-in-oil-in-water type double emulsion (w/o/w); and
(c) solidifying the double emulsion of step (b).

In the method for producing nanoparticles of the present invention, the "first water phase" may be an aqueous solvent (pH 7.0 to 8.0) containing a calcium carbonate slurry, specifically, for example, distilled water in which calcium carbonate crystals are suspended, physiological saline (PBS), an aqueous surfactant solution (aqueous PVA solution), or the like. The "oil phase" in step (a) of the present invention may be a phase in which a fatty acid and a biocompatible polymer is dissolved in a hydrophobic and highly volatile organic solvent immiscible with a water phase, specifically, for example, a phase in which a fatty acid and a biocompatible polymer is dissolved in methylene chloride, chloroform, dimethylformamide, ethyl acetate, acetone, acetonitrile, tetrahydrofuran, dimethyl sulfoxide, and mixed solvents thereof. The mixing of the first water phase and the oil phase in step (a) of the present invention may be preferably carried out using mechanical stirring, for example, an ultrasonic crusher, a homomixer, a stirrer, or the like.

The single emulsion (w/o) prepared through step (a) of the present invention is mixed with the second water phase to form the double emulsion (w/o/w). Specifically, the second water phase may be, for example, an aqueous surfactant solution such as polyvinyl alcohol, or poloxamer, polyvinylpyrrolidone. The resulting double emulsion can be solidified through evaporation or extraction of an organic solvent.

In one embodiment of the present invention, the weight ratio of the fatty acid to the biocompatible polymer in the single emulsion of step (a) of the present invention may be 0.01:1 to 0.2:1 (fatty acid: polymer), preferably 0.05:1 to 0.15:1. In addition, the weight ratio of the calcium carbonate crystal to the biocompatible polymer may be 0.001:1 to 1:1 (calcium carbonate: polymer), preferably 0.01:1 to 0.8:1, more preferably 0.1:1 to 0.6:1.

The nanoparticle manufacturing method according to the present invention is concerned with a method for producing the nanoparticles, which is another aspect of the present invention, and the contents overlapping with the nanoparticles are omitted in order to avoid excessive complexity of description in this specification.

### [Advantageous Effects]

The polymer nanoparticles according to the present invention are specifically introduced into adipocytes while circulating in the body and generate carbon dioxide within the cells, thereby decomposing fat through necrosis of the adipocytes. In particular, according to the present invention, since targeting to the adipocytes is possible by using a ligand (fatty acid), the effect on surrounding tissues and cells can be minimized, thereby developing a product that can minimize the side effects of drugs and enable safer procedures. The nanoparticles can be applied to areas such as chin, thighs, arms, and stomach, which are generally treated with high frequency.

In addition, the nanoparticles according to the present invention can be produced as an injectable preparation, and can be applied in the field of diet beauty and obesity treatment and used as local lipolysis supplements, body shape corrections, or diet beauty products that decompose local fat through its necrosis.

### [Description of Drawings]

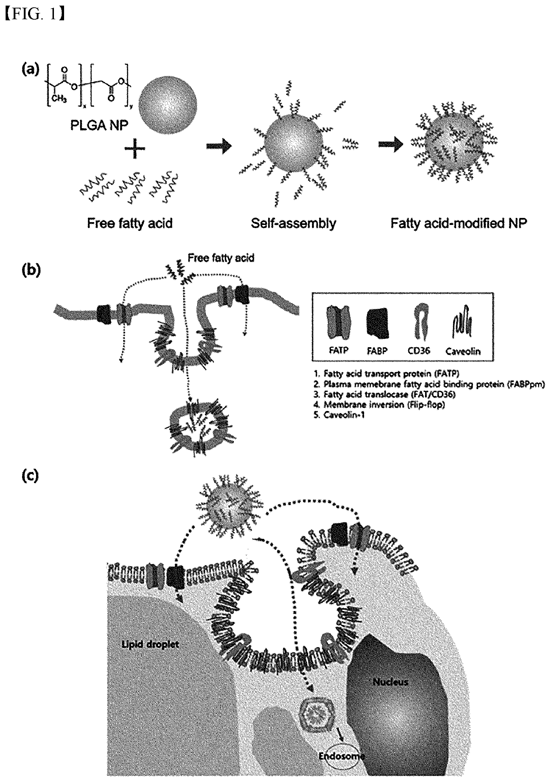

FIG. 1 is a schematic diagram showing fatty acid-introduced nanoparticles (a) and an endocytosis process of fatty acid (b) and fatty acid-introduced nanoparticles (b) into adipocytes according to an example of the present invention.
FIG. 2 is a diagram showing the size distribution of (a) PNP, (b) GNP, (c) PA-GNP2, and (d) PA-GNP10 measured by DLS.
FIG. 3 shows SEM images of (a) PNP, (b) GNP, (c) PA-GNP2 and (d) PA-GNP10.
FIG. 4 shows the 1H NMR spectrum of nanoparticles for each concentration of palmitic acid.
FIG. 5 shows the py-GC/MS spectrum of nanoparticles for each concentration of palmitic acid.
FIG. 6 shows the in vitro viability of cells treated with GNP, PA-GNP2 and PA-GNP10 ([CaCO3] = 0.5 mg/mL).
FIG. 7 shows oil red O staining images of (a) 3T3-L1 preadipocyte, (b) 3T3-L1 adipocyte on day 6, (c) 3T3-L1 adipocyte on day 10, (d) 3T3-L1 adipocyte on day 12 after culture, and (e) changes in the optical density values of oil red O eluted from the 3T3-L1 preadipocyte and adipocytes over time.
FIG. 8 shows the in vitro viability of 3T3-L1 adipocytes treated with various concentrations of PNP, GNP, PA-PNP2 and PA-PNP10.
FIG. 9 shows (a) the in vitro viability of 3T3-L1 adipocytes treated with GNP, PA-GNP2 and PA-GNP10 ([CaCO3] = 0.5 mg/mL) and the in vitro viability of 3T3-L1 adipocytes treated with PA-GNP10 containing various concentrations of CaCO3.
FIG. 10 shows confocal microscopy images of 3T3-L1 adipocytes treated with PNP and PA-PNP conjugated with Alexa 488 cadaverine.
FIG. 11 shows confocal microscopy images of 3T3-L1 adipocytes treated with PNP and various types of FA-PNP conjugated with Alexa 488 cadaverine.

### [Best Modes of the Invention]

Hereinafter, the present invention will be described in more detail by way of examples. These examples are only for illustrating the present invention more specifically, and it will be apparent to those skilled in the art that the scope of the present invention according to the subject matter of the present invention is not limited by these examples.

### EXAMPLES

### Reference experimental materials

Poly(DL-lactide-co-glycolide) (PLGA, Mw 6400, 50:50, 0.15-0.25 dL/g, carboxylate end group) was purchased from Lactel Absorbable Polymers (Birmingham, AL, USA). Poly(vinyl alcohol) (PVA, Mw 30000-70000, 87-90 % hydrolyzed), dichloromethane (methylene chloride, MC), calcium carbonate (CaCO₃), palmitic acid (PA), oil red O, sodium hydroxide (NaOH), hydrochloride (HCl), sodium acetate (anhydrous), acetic acid, dimethylsulfoxide (DMSO), 3-isobutyl-1-methyl-xanthine (IBMX), insulin, dexamethasone, Dulbecco's phosphate buffered saline (DPBS), penicillin-streptomycin glutamine, and 10% formalin solution were purchased from Sigma-Aldrich (St. Louis, MO, USA). 2-propanol (isopropanol, 99.5%) was purchased from Samcheon (Gangnam, Seoul, Korea). CellTiter 96^{®} Aqueous One Solution (MTS assay) was purchased from Promega (Madison, WI, USA). Dulbecco's modified Eagle's medium (DMEM, 4.5 g/L, D-glucose) was purchased from Wellgene (Gyeongsan, Gyeongbuk, Korea). Fetal bovine serum (FBS) and bovine calf serum (CS) were purchased from Gibco (Grand Island, NY, USA). Trypsin-ethylenediaminetetraacetic acid and Hoechst 33342 were purchased from Thermo Fisher Scientific (Waltham, MA, USA). Alexa fluor^{™} 488 cadaverine sodium salt and Lysotracker red DND-99 were purchased from Invitrogen (Eugene, OR, USA). VECTASHIELD^{®} Antifade mounting medium was purchased from Vector Laboratories (Burlingame, CA, USA). Water was distilled and deionized using a Milli-Q (R) System (Millipore; Billerica, MA, USA) and water purification RO MAX (Human Science; Hanam, Gyeonggi, Korea). Mouse C57BL/6 was purchased from Orient (Seongnam, Gyeonggi, Korea).

### Example 1. Preparation of gas-generating nanoparticles (PA-GNPs) modified with palmitic acid

Fatty acid-modified gas-generating PLGA nanoparticles (FA-GNPs) were prepared by water-in-oil-in-water (W/O/W) double emulsion and solvent evaporation methods. The fatty acids physically modified the GNP surface. A fatty acid solution dissolved in 10 % w/v dichloromethane was mixed with a PLGA solution (5 % w/v) for at least 4 hours (FA-PLGA solution). (FA-PLGA solution, 25% w/v calcium carbonate (included) in deionized water and 4% PVA solution were fixed in ice bath until used.) The calcium carbonate (CaCO₃) was evenly dispersed with deionized water (DW) using a sonicator (Branson Digital Sonifier^{®}; Danbury, CT, USA) for 90 seconds at 25 W output (W₁). In order to load CaCO₃ into nanoparticles, the CaCO₃ dispersion solution was added to FA-PLGA solution (O) and emulsified with a sonicator at 25 W output for 90 seconds. This emulsion (W₁/O) was poured into a 4% PVA solution used as a suspension polymerization aid and was emulsified with the sonicator at 35 W output for 2 minutes to make another emulsion (W₁/O/W₂). The complete double emulsion was added to the 1% PVA solution and stirred overnight to evaporate the remaining dichloromethane. Unloaded CaCO₃ was removed by ultracentrifugation at 4000 g for 1 min. Thereafter, the supernatant was ultracentrifuged at 31,000 g for 30 minutes to collect nanoparticles, which were washed four times with deionized water. FA-GNP was freeze-dried (Ilshin Biobase Freeze Dryer, Dongducheon, Gyeonggi, Korea) and then stored at 4°C. FA-PLGA nanoparticles (FA-PNP) without CaCO₃ were prepared in the same manner and used as a control.

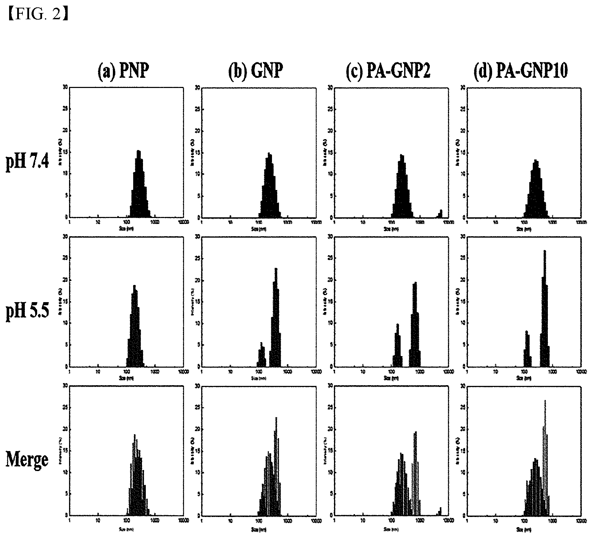

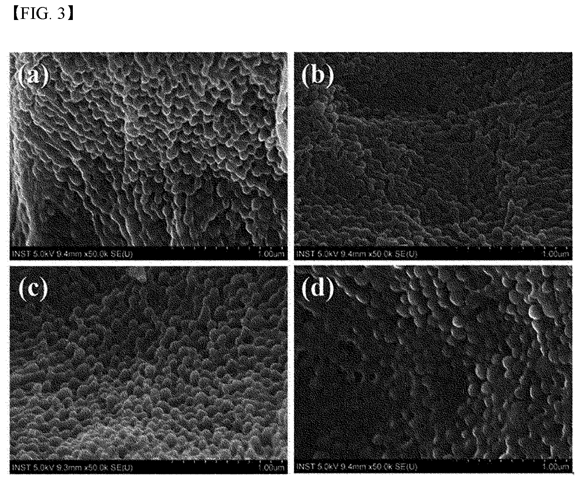

PLGA nanoparticles containing calcium carbonate were prepared by a W/O/W double emulsification method and used as gas-generating nanoparticles (GNPs). PLGA nanoparticles containing no calcium carbonate (PNP, no gas generation) were also prepared and used as a control. When the loading content of CaCO₃ in the GNP was 55.0% (CaCO₃/PLGA; wt/wt%), the average diameter of the GNPs was 246.8 nm. No significant change in the average diameter of PNPs according to changes in the calcium carbonate content was observed (Table 1). This appeared at a polydispersity index (PDI) value of 2.5, and a narrow size distribution was showed under neutral pH conditions (pH 7.4) (FIGS. 2a and 2b). The SEM images showed that the nanoparticles formed a spherical shape even in the presence of CaCO₃ (FIGS 3a and 3b). The pH-dependent size distribution change was measured as follows. The PNP did not show any difference in size according to pH. Interestingly, it was confirmed that under acidic conditions (pH 5.5), GNP was not only greatly increased in size but also broadened in size distribution (FIG. 2). This result may suggest that the GNPs generate carbon dioxide gas in response to acidic pH conditions.

**[Table 1]**

| **Sample** | **Feed ratio** | **Loading content** | **Mean diameter (nm)** | **PDI** |
|---|---|---|---|---|
| | **PA/PLGA (wt %)** | **CaCO₃/PLGA (wt %)** | | |
| PNP | 0 | - | 249.3 ± 2.3 | 0.115 |
| PA-PNP2 | 2 | - | 246.3 ± 1.4 | 0.167 |
| PA-PNP10 | 10 | - | 254.9 ± 0.6 | 0.164 |
| GNP | 0 | 55.0 | 246.8 ± 2.7 | 0.227 |
| PA-GNP2 | 2 | 44.0 | 245.3 ± 2.7 | 0.240 |
| PA-GNP10 | 10 | 43.8 | 252.8 ± 4.7 | 0.183 |

### Experimental Example 1. Characteristics of gas-generating nanoparticles (PA-GNPs) modified with palmitic acid

The morphology of nanoparticles was observed with a scanning electron microscope (SEM) (S-4800 U field emission scanning electron microscope, Hitachi, Tokyo, Japan). The average size and size distribution of PA-GNPs were determined by dynamic light scattering (DLS) (Nano ZS, Malvern instrument, UK) at pH 7.4 (deionized water) and pH 5.5 (acetic acid) ([NPs]=0.5 mg/ml). NMR and py-GC/MS were used for the qualitative analysis of palmitic acid. NMR spectroscopy was used to obtain the spectrum. All samples were diluted to an equivalent concentration of 2 mg/mL using DMSO as a solvent. The spectrum was also obtained using py-GC/MS and 4 mg was used for all samples. To determine the loading content of CaCO₃, the PA-GNP was dissolved in 1M NaOH solution for 1 hour and neutralized with 1M HCl solution. The concentration percentage of CaCO₃ in the nanoparticle solution was estimated using a calcium colorimetric analysis kit from Biovision (Palo Alto, CA, USA) and calculated as a weight fraction. The amount of carbon dioxide gas generated from PA-GNP can be measured with a calcium colorimetric analysis kit and can be calculated by determining the number of moles of calcium ions (Ca²⁺/CO₂=1 mol/mol). The nanoparticles were suspended in phosphate buffer (pH 7.4 or pH 5.5, [CaCO₃] = 1 mg/mL) and the solution was ultracentrifuged at 31,000 g for 15 min.

Palmitic acid (PA), a saturated fatty acid commonly found in animals, was selected and used for surface modification of the PLGA nanoparticles because it is less sensitive to light, air and heat than oleic acid, another fatty acid abundant in animals. The PA was physically bound to the PLGA nanoparticles in various weight ratios for enhanced adipocyte uptake. The palmitic acid-modified gas-generating PLGA nanoparticles (PA-GNP) were prepared by self-assembly between the PA and the PLGA nanoparticles in the O phase during the emulsification process. The number after the PA-GNP indicates the feed ratio of the palmitic acid and the PLGA (Table 1). The PLGA itself and unmodified nanoparticles (GNPs) were used as controls.

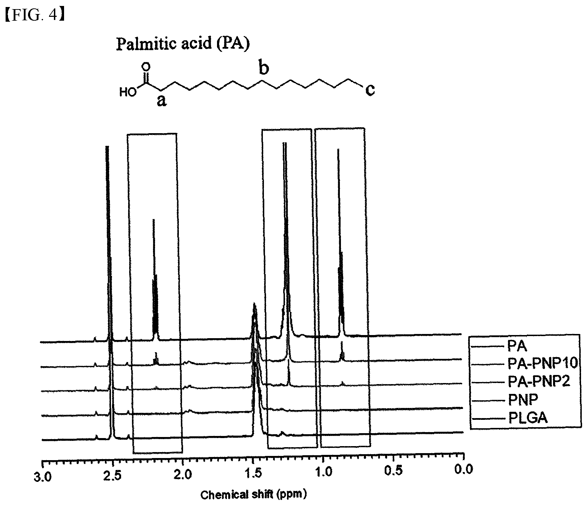

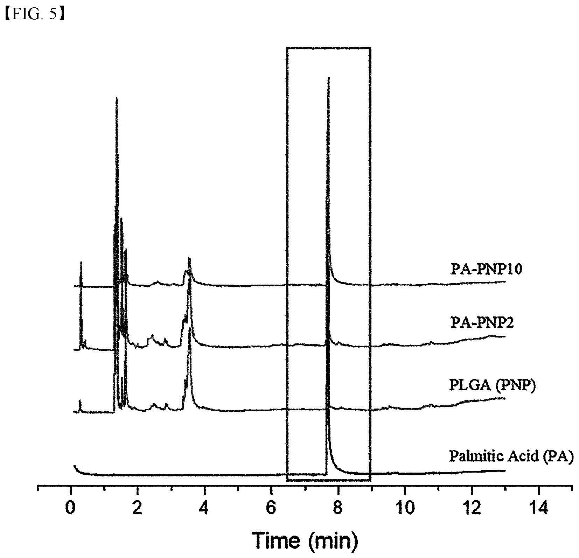

With the addition of the palmitic acid and the calcium carbonate, there was no significant change in the average diameter of the nanoparticles according to the amount of the palmitic acid added (Table 1). The amount of the PA attached to the surface was qualitatively analyzed through NMR and py-GC/MS. Peaks at 5 and 1.5 ppm in the PNP and PA-PNP spectra were identified as PLGA. In the case of PA-PNP, new peaks corresponding to the PA were observed at 2.2 ppm (-O-CO-CH₂, -COOH near CH₂), 1.35 ppm (-(CH₂)n-) and 0.85 ppm (-CH₃) (FIG. 4). As the feed ratio of fatty acids increased, the surface modification efficiency decreased (Table 2). It was assumed that the low temperature (10°C) during the ultracentrifugation process would affect the fatty acid loss. Based on the palmitic acid peak observed between 7.5 and 7.8 minutes, it was confirmed that the corresponding peak did not appear in the PNP at 7.5-7.8 minutes (FIG. 5). In addition, in the PA-PNP to which the palmitic acid was added, the degree of the peak was changed according to the concentration of palmitic acid. The palmitic acid peak area was calculated as an integral value to calculate the amount of the palmitic acid attached to the nanoparticles. Considering the loss of palmitic acid during the nanoparticle manufacturing process, it was found that the amount of palmitic acid actually differed by 6.59 times, not 5 times. These ¹H NMR and py-GC/MS results indicated that the PA was successfully bound to the nanoparticle surface.

The morphology of PA-PNP was also observed by scanning electron microscopy. Even when the palmitic acid was added, the PA-PNP was still spherical compared to the PNP and there was no significant change in size (FIGS. 3c and 3d). The size change under acidic conditions was similar to that of the GNP even when the palmitic acid was attached. Through the above results, it was confirmed that the surface modification by palmitic acid did not significantly affect the physical properties such as the size, shape, and gas-generating ability of the GNP.

**[Table 2]**

| **Sample** | **Feed ratio** | **Actual amount Concentration of** |
|---|---|---|
| | **PA/PLGA; wt %** | **PA/PLGA; wt %** |
| PNP | 0 | 0 |
| PA-PNP2 | 2 | 1.7 |
| PA-PNP10 | 10 | 5.3 |

### Experimental Example 2. Analysis of viability of nanoparticle-treated cells Cell culture and 3T3-L1 adipocyte differentiation

NIH-3T3 cells (fibroblasts) were purchased from American Type Culture Collection (ATCC, Manassas, VA, USA) and the cells were maintained in DMEM complete growth medium containing 10% CS and 1% penicillin and streptomycin (PS) under 37°C and 5% CO₂ conditions (10% CS medium). C2C12 cells (myocytes) were purchased from ATCC and maintained in DMEM complete growth medium containing 10% FBS and 1% PS (10% FBS medium) at 37°C under 5% CO₂ conditions. 3T3-L1 preadipocytes were purchased from ATCC and maintained in 10% CS medium at 37°C under 5% CO₂ conditions. Two days after 3T3-L1 preadipocytes reached confluency, the cells were cultured for 2 days in a growth medium (MDI medium) containing 1% IBMX (0.5 mM), 0.1% insulin (1 µg/mL), and 0.1% dexamethasone (1 µM) mixed in 10% FBS to initiate adipocyte differentiation. The cells were cultured for 2 days in DMEM medium containing 10% FBS and 0.1% insulin. Finally, the differentiated cells were cultured in DMEM medium containing 10% FBS, and the medium was replaced every other day.

### Oil Red O staining

Oil Red O staining was used to determine the degree of adipocyte differentiation and to indicate lipid droplets. 0.7 g of Oil Red O powder was dissolved in 200 mL of isopropane, stirred overnight, filtered through a 0.22 µm syringe filter, and stored at 4°C to prepare an Oil Red O stock solution. The stock solution and deionized water were mixed at a ratio of 3:2 to prepare an Oil Red O working solution, which was left at room temperature (RT) for 20 minutes, and then filtered through a 0.22 µm syringe filter before used. The cells were washed with 10% formalin for 5 min at RT and fixed with 10% formalin for 1 h at RT. After fixed, the cells were washed with 60% isopropanol and dried completely. Then, the Oil Red O working solution was added for 10 minutes. The cells were washed at least 4 times with deionized water to remove residues of the Oil Red O stock solution. After photographed under a microscope, it was completely dried again. The Oil Red O was pipetted up and down several times and eluted with 100% isopropanol, then measured at 500 nm using an ultraviolet/visible spectrophotometer (SpectraMax ABS, Molecular Devices, San Jose, CA, USA).

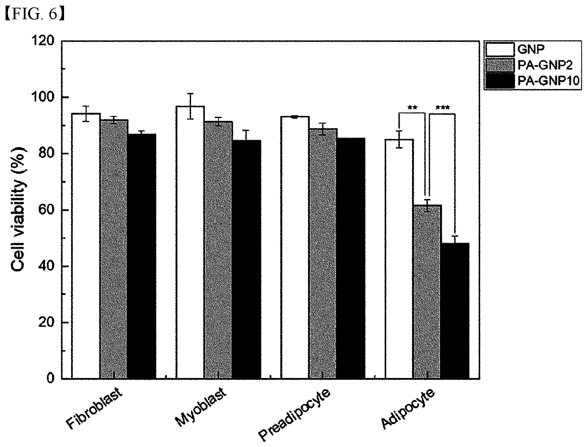

To confirm the delivery ability of the PA-GNP and the effect of in vitro treatment according to it, viability experiments were analyzed and evaluated by MTS for various cell lines. NIH-3T3 fibroblasts, C2C12 myoblasts, 3T3-L1 preadipocytes, and 3T3-L1 adipocytes were used as competition groups (FIG. 6). Since most cells absorb fatty acids and use them as an energy source, it was attempted to confirm how specifically they are absorbed by the adipocytes.

The GNP did not affect the cell viability of all other cell lines such as adipocytes, preadipocytes, stem cells, fibroblasts and myoblasts. This suggests that the cells cannot absorb only GNP and that carbon dioxide gas production does not occur at biological pH. Interestingly, when the PA-GNP was used, the presence of the PA did not significantly affect other cell lines, but in the case of adipocytes, the viability was particularly reduced. Based on these results, it was found that the PA-GNP is effective in adipocytes, where fatty acids are most absorbed. In addition, it was confirmed that the acidic state of endocytosis generated in the process of absorbing fatty acids promotes carbon dioxide gas production and greatly reduces cell viability.

### Experimental Example 3. Analysis of specific absorption of nanoparticles into adipocytes and ability of nanoparticles to kill adipocytes

The absorption of PA-PNP into 3T3-L1 adipocytes was confirmed by a confocal laser scanning microscope (TCS SP5, Leica Microsystems, Germany). First, Alexa fluor^{™} 488 cadaverine was dissolved in MES buffer and reacted with PA-PNP overnight under dark conditions to prepare Alexa 488 cadaverine-PA-PNPs (Alexa 488 cadaverine/polymer=1/50, w/w). The labeled nanoparticles were collected by ultracentrifugation at 31,000 g for 15 minutes, and washed three times with deionized water. The Alexa 488 Cadaverine-PA-PNP was lyophilized and then stored at 4°C.

3T3-L1 preadipocytes were seeded in confocal dishes at the recommended seeding density of 8×10⁴ cells and differentiated as described in the section above. The adipocytes were treated with a medium containing Lysotracker red (50 nM) and Alexa 488 cadaverine-PA-PNP (0.5 mg/mL) for 24 hours. Thereafter, the adipocytes were washed three times with DPBS to remove the remaining nanoparticles, and were treated with HOECHST (1 µg/mL) for 15 minutes to stain the nuclei. The cells were fixed with 10% formalin for 15 min, mounted using an antifade mounting medium (VECTASHIELD^{®}, Vector Laboratories, Burlingame, CA, USA), and analyzed by a confocal microscope. All processes were performed under dark conditions.

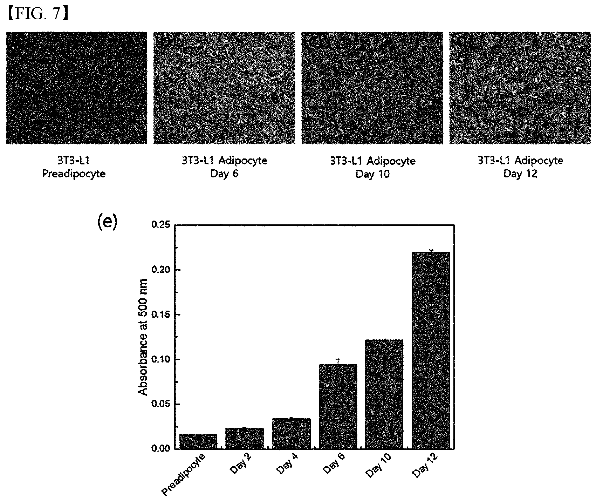

3T3-L1 preadipocytes were cultured in CS medium and differentiated into adipocytes in FBS medium. After differentiation into adipocytes, lipid droplets were observed in the cytoplasm and detected by Oil Red O staining (FIG. 7). The accumulation of the lipid droplets was investigated step by step from before differentiation to 12 days after differentiation.

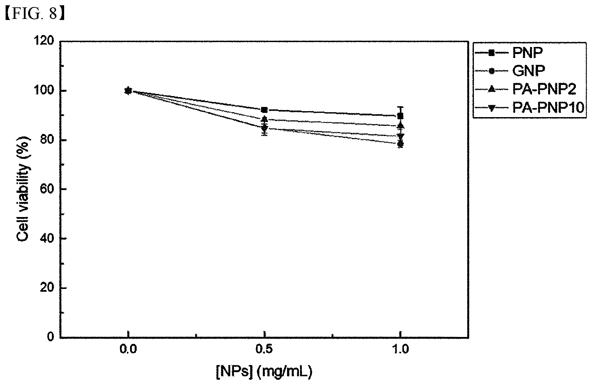

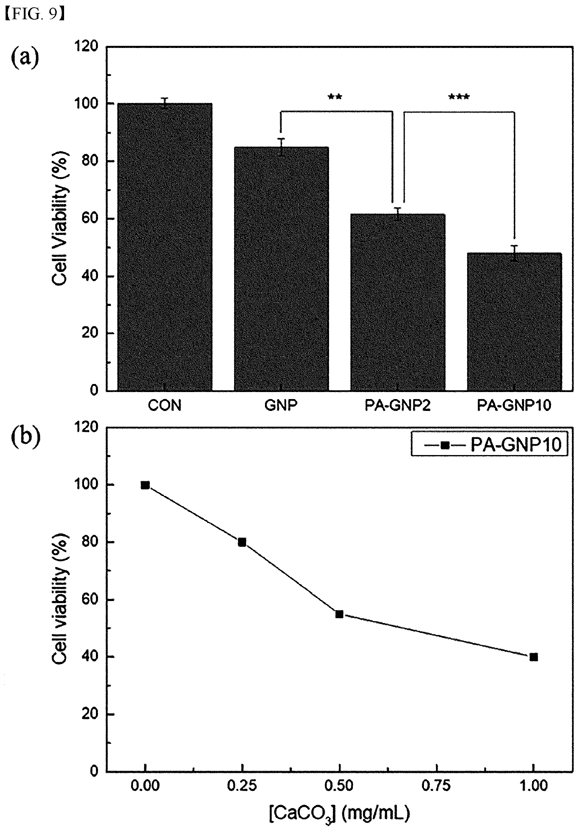

The cytotoxicity of nanoparticles was evaluated by MTS analysis of 3T3-L1 adipocytes. As a control, untreated cells were used, and cells treated with PNP, GNP, PA-PNP2 and PA-PNP 10 did not show a significant change according to the sample concentration (FIG. 8). This may also suggest that PLGA, PA and CaCO₃ used for nanoparticle production have no appreciable toxicity. On the other hand, as the concentration of PA increased, the cell viability of the cells treated with the PA-GNP decreased (FIG. 9). This was considered to be of important significance and was used for further experiments. Although not shown in the drawings, the cell viability tended to be the lowest when the PA/PLGA ratio was 1, but the nanoparticles were excluded from the experimental group because they were too oily to be tested.

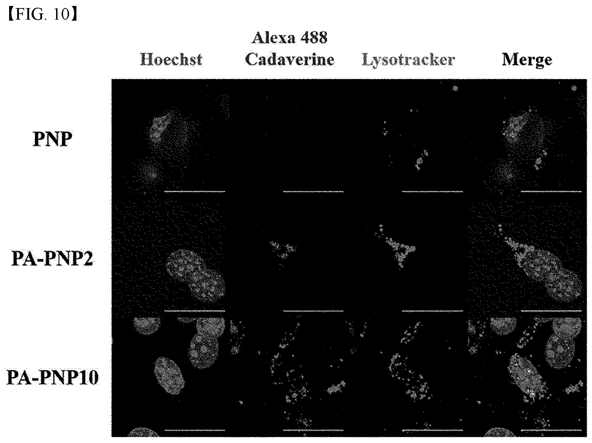

Specific cellular absorption of the PA-PLGA nanoparticles into 3T3-L1 adipocytes was examined by a confocal microscope using an Alexa 488 cadaverine-PA-PNP. Cellular absorption of the PA-PNP into 3T3-L1 adipocytes was clearly observed compared to nanoparticles whose surface was not modified (FIG. 10). The Lysotracker probe used in this experiment has a weak base that is partially neutralized at neutral pH and can freely penetrate the cell membrane. It is also highly selective for organelles with acidity. Therefore, in this experiment, lysosomes representing the acidic pH inside the cells are selectively stained and appear red. As assumed in this study, the nanoparticles whose surface is modified with the green label of Alexa 488 cadaverine are absorbed by various mechanisms to enter endosomes, eventually leading to co-localization with lysosomes. Therefore, when the lysosomes and the nanoparticles meet, appearance of yellow indicates successful absorption. In addition, it can also be predicted that carbon dioxide gas is generated in the PA-GNP in the future to kill adipocytes.

### Experimental Example 4. Analysis of absorption capacity of nanoparticles modified by various fatty acids into adipocytes

CaCOs-free FA-PLGA nanoparticles (FA-PNP) were prepared using fatty acids having various lengths of carbon chains in the same manner as in Example 1 above to evaluate whether the endocytosis into adipocytes occurs.

Specifically, myristic acid (MA, C14), palmitic acid (PA, C16) and stearic acid (SA, C18), which are saturated fatty acids, and oleic acid (OA, C18), which is an unsaturated fatty acid, were used, and the fatty acids and the PLGA were used in the ratio shown in Table 3 below to prepare FA-PLGA nanoparticles (FA-PNP).

**[Table 3]**

| **Sample** | **Feed ratio** |
|---|---|
| | FA/PLGA (wt %) |
| PNP | 0 |
| PA-PNP 10 | 10 |
| MA-PNP 10 | 10 |
| SA-GNP 10 | 10 |
| OA-GNP 10 | 10 |

The absorption capacity of the prepared FA-PNP into adipocytes was analyzed in the same manner as in Experimental Example 3.

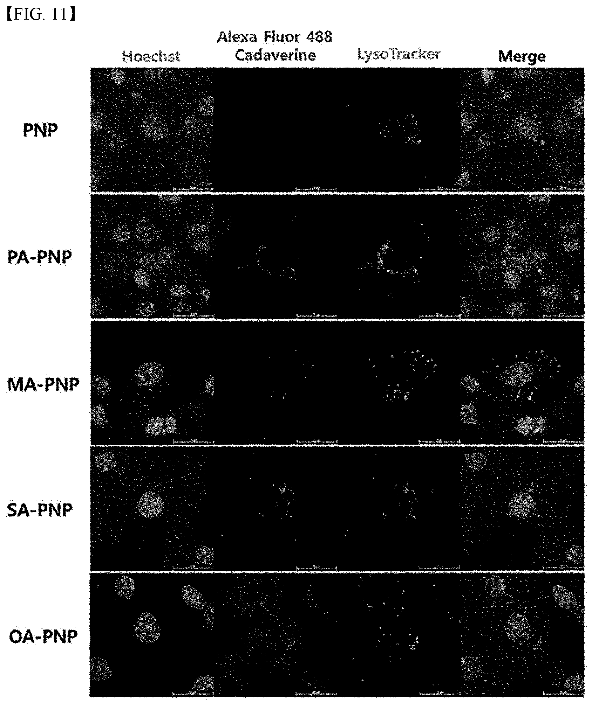

As a result, as shown in FIG. 11, it was confirmed that the absorption capacity of the nanoparticles into adipocytes was improved even when the saturated and unsaturated fatty acids having different carbon numbers as well as palmitic acid were used.

## Claims

1. A nanoparticle comprising: a calcium carbonate crystal, a biocompatible polymer and a fatty acid, wherein the calcium carbonate is embedded in the nanoparticle, and the fatty acid is exposed on the surface of the nanoparticle.

2. The nanoparticle according to claim 1, wherein the nanoparticle releases carbon dioxide in an acidic environment.

3. The nanoparticle according to claim 1, wherein the biocompatible polymer is a polymer having a polylactide (PLA), polyglycolide (PGA), polylactide-polyglycolide copolymer (PLGA), starch, glycogen, chitin, peptidoglycan, lignosulfonate, tannic acid, lignin, pectin, polyethylene glycol, polyethylene oxide, polyvinyl alcohol, polyethylene oxide-polypropylene oxide block copolymer, cellulose, hemicellulose, heparin, hyaluronic acid, dextran or alginate structure.

4. The nanoparticle according to claim 1, wherein the nanoparticle has a diameter of 100 to 300 nm.

5. The nanoparticle according to claim 1, wherein the fatty acid is a saturated fatty acid or an unsaturated fatty acid.

6. The nanoparticle according to claim 1, wherein the weight ratio of the fatty acid and the biocompatible polymer is 0.01: 1 to 0.2:1.

7. A composition for lipolysis, comprising the nanoparticle according to any one of claims 1 to 6.

8. A pharmaceutical composition for preventing or treating obesity, comprising the composition for lipolysis according to claim 7.

9. A method for producing the nanoparticle of any one of claims 1 to 6, comprising the steps of:
(a) mixing a first water phase containing calcium carbonate crystals with an oil phase containing fatty acids and biocompatible polymers to form a water-in-oil type single emulsion (W/O);
(b) mixing the emulsion of step (a) with a second water phase to form a water-in-oil-in-water type double emulsion (W/O/W); and
(c) solidifying the double emulsion of step (b).

10. The method according to claim 1,
The method according to claim 9, wherein the biocompatible polymer is a polymer having a polylactide (PLA), polyglycolide (PGA), polylactide-polyglycolide copolymer (PLGA), starch, glycogen, chitin, peptidoglycan, lignosulfonate, tannic acid, lignin, pectin, polyethylene glycol, polyethylene oxide, polyvinyl alcohol, polyethylene oxide-polypropylene oxide block copolymer, cellulose, hemicellulose, heparin, hyaluronic acid, dextran or alginate structure.

11. The method according to claim 9, wherein the diameter of the nanoparticle produced by the method is 100 to 300 nm.

12. The method according to claim 9, wherein the fatty acid is a saturated fatty acid or an unsaturated fatty acid.

13. The method according to claim 9, wherein the biocompatible polymer and the fatty acid are mixed in a weight ratio of 0.01:1 to 0.2:1.
